# EUROPEAN PATENT APPLICATION

(11) **EP 3 524 691 A1**
(43) Date of publication of application: **14.08.2019**
(21) Application number: 19150175.8
(22) Date of filing: 06.12.2013
(51) Int. Cl.: C12Q 1/68, C12Q 1/6883

(54) **STRATIFICATION AND TREATMENT OF PATIENTS OF IDIOPATHIC THROMBOCYTOPENIC PURPURA**

(30) Priority: 07.12.2012 EP 12008209
(62) Divisional of application: 13196101.3
(71) Applicant: SuppreMol GmbH, 82152 Martinsried (DE)
(72) Inventor: Sondermann, Peter, 82131 Stockdorf (DE); Ter Meer, Dominik, 81377 München (DE)
(74) Representative: Hoffmann Eitle

(57) **Abstract**

The present invention provides for a method for excluding a non-rheumatic disorder as cause of a musculoskeletal complaint of a subject comprising determining as to whether at least one autoantibody or antigen which is indicative for at least one rheumatic disorder is present in a sample obtained from the subject, wherein the presence of said autoantibody or antigen allows to exclude a non-rheumatic disorder as cause of a musculoskeletal complaint of a subject. Accordingly, it provides for a method that triggers the next steps in diagnosing the cause of a musculoskeletal complaint of a subject - either further diagnosis of a rheumatic disorder or of a non-rheumatic disorder to confirm the initial diagnosis. Also provided is a kit for excluding a non-rheumatic disorder as cause of a musculoskeletal complaint of a subject as well as uses of the kit.

## Description

### BACKGROUND

Idiopathic thrombocytopenic purpura (ITP) is the condition of having an abnormally low platelet count (thrombocytopenia). As most incidents of ITP appear to be related to the production of antibodies against platelets, immune thrombocytopenic purpura or immune thrombocytopenia are terms also used to describe this condition. Often ITP is asymptomatic and can be discovered incidentally, but a very low platelet count can lead to an increased risk of bleeding and purpura.

ITP can be diagnosed with a complete blood count (a common blood test). In some situations, additional investigations (such as a bone marrow biopsy) may be necessary to ensure that the platelet count is not decreased due to other reasons. Treatment may not be necessary in mild cases, but very low counts or significant bleeding might prompt treatment with steroids, intravenous immunoglobulin, anti-D immunoglobulin, or stronger immunosuppressive drugs. Refractory ITP (ITP not responsive to conventional treatment) may require splenectomy, the surgical removal of the spleen. Platelet transfusions may be used in severe bleeding together with a very low count.

An individual exhibiting a platelet count below 20,000 per µl is generally an indication for treatment. The first line of treatment usually consists of steroids. The dose and mode of administration is determined by the platelet count and whether there is active bleeding. In emergencies, infusions of dexamethasone or methylprednisolone may be used, while in milder forms the treatment may consist of oral prednisone or prednisolone. Once the platelet count has improved, the dose of steroid is gradually reduced while monitoring for relapses. Another strategy that is suitable for Rh-positive patients is treatment with Rho(D) immune globulin (Anti-D), which is intravenously administered. Anti-D is normally administered to Rh-negative women during pregnancy and after the birth of an Rh-positive infant to prevent sensitization to the Rh factor in the newborn. Anti-D has been demonstrated effective on some ITP patients, but is costly, produces a short-term improvement and is not recommended for post-splenectomy patients.

Another treatment strategy involves the use of thrombopoietin receptor agonists. They are pharmaceutical agents that treat ITP by stimulating platelet production instead of attempting to curtail platelet destruction. Currently two such products are available on the market. Romiplostim (trade name Nplate) is a thrombopoiesis stimulating Fc-peptide fusion protein (peptibody) that is administered by subcutaneous injection. Clinical trials have demonstrated romiplostim to be effective in treating chronic ITP, especially in relapsed post-splenectomy patients. Romiplostim was approved by the United States Food and Drug Administration (FDA) for long-term treatment of adult chronic ITP in 2008. On the other hand, Eltrombopag (trade name Promacta in the USA, Revolade in the EU) is an orally-administered agent with an effect similar to that of romiplostim. It too has been demonstrated to increase platelet counts and decrease bleeding in a dose-dependent manner.

Another drug that is presently under clinical investigatins where it shows very promising results is SM101, a soluble Fc gamma receptor IIB (sFcγRIIB), which competes with FcγRs expressed on immune cells for pathogenic immune complexes. Interference at this early stage of the immune reaction prevents the triggering of the cascade that results in inflammation and tissue destruction. In addition to immune complex competition, SM101 interferes with the formation of memory/plasma cells that are causative for the formation of new pathogenic antibodies. This dual mode of action strongly suggests SM101's potential for the treatment of different autoimmune diseases. SM101 has been extensively profiled in in-vitro, ex-vivo and in-vivo experiments in order to confirm its mechanisms of action in a broad range of preclinical studies. SM101 was also tested in a range of animal disease models with a predictive value for future clinical outcome in patients. To date, SM101 has been tested in ITP patients and has shown excellent safety data and long lasting pharmacodynamic effects. Of course, at times where personalized or individually tailored medicine becomes more and more important in order to achieve an as good as possible therapeutic result for patients suffering from a disease, it is always desirable to know and/or understand beforehand and/or sometimes during treatment as to whether a patient may or may not respond to a therapy. Accordingly, it is a desire to have available means and methods that put a practitioner as well as patients into a position to understand and/or know as to whether patients may or may not respond to a therapy. With this goal in mind, the technical problem underlying the present invention is the provision of means and methods for predicting a potential therapeutic effect to Fc gamma receptor IIB (FcγRIIB) therapy and/or stratifying patients with regard to their susceptibility to treatment with a Fc gamma receptor IIB (FcγRIIB) therapy.

### SUMMARY

The technical problem was solved by the present inventors in that they have observed that the allelic pattern for the Fc gamma receptor IIB (FcγRIIB) of an individual is a predictive marker for a therapeutic response to Fc gamma receptor IIB (FcγRIIB) therapy. In particular, it was surprisingly found that the presence of the homozygous FcγRIIB 232 T/T genotype is found to be indicative of an individual that will exhibit a negative therapeutic response to said Fc gamma receptor IIB (FcγRIIB) therapy, whereas the presence of the homozygous FcγRIIB 232 I/I genotype is found to be indicative of an individual that will exhibit a positive therapeutic response to said Fc gamma receptor IIB (FcγRIIB) therapy (see the Table below).

**FcgRIIB 232**

| Genotype | % Responder | % Non-Responder |
|---|---|---|
| AA II | 80% | 0% |
| GG TT | 0% | 20% |

It is assumed that also the heterozygous FcγRIIB 232 I/T genotype is indicative of an individual that will exhibit a positive therapeutic response to said Fc gamma receptor IIB (FcγRIIB) therapy, i.e. all embodiments disclosed herein which relate to the homozygous FcγRIIB 232 I/I genotype are likewise valid for heterozygous FcγRIIB 232 I/T genotype.

It is also assumed that patients having other B-cell mediated diseases apart from ITP can be stratified/treated in accordance with the teaching of the present invention. Other B-cell mediated diseases apart from ITP are preferably autoimmune diseases such as Multiple Sclerosis, rheumatoid arthritis and/or Systemic lupus erythematosus (SLE).

Seven non-synonymous SNPs have been identified in human FCGR2B, but only one of these SNPs has been shown to occur at notable frequency. This non-synonymous T-to-C transition in exon 5 (rs1050501) results in the substitution of a threonine for isoleucine at position 232 within the transmembrane domain of FcγRIIB (referred to as FcγRIIBT232). The FcγRIIBT232 variant is excluded from lipid rafts, leading to reduced phosphorylation by LYN, abnormal SHIP recruitment and a decrease in FcγRIIB-mediated inhibition of both macrophage and B cell responses.
However, this genotype-phenotype correlation provides no evidence or hint whatsoever that it could serve as a marker for predicting a therapeutic response to Fc gamma receptor IIB (FcγRIIB) therapy in an individual suffering from Idiopathic thrombocytopenic purpura (ITP). In fact, without being bound by theory the Fc gamma receptor IIB (FcγRIIB) therapy, in particular the application of SM101, a soluble Fc gamma receptor IIB (sFcγRIIB), is thought to compete with FcγRs expressed on immune cells for pathogenic immune complexes. As such, it could not have been reasonably expected that the allelic pattern at position 232 within the transmembrane domain of FcγRIIB may be useful as a marker for predicting a therapeutic response to Fc gamma receptor IIB (FcγRIIB) therapy in an individual suffering from Idiopathic thrombocytopenic purpura (ITP).

Accordingly, given the surprising finding of the present inventors, the present invention provides a novel method for predicting therapeutic response to Fc gamma receptor IIB (FcγRIIB) therapy in an individual suffering from Idiopathic thrombocytopenic purpura (ITP) by detecting the presence of the allelic pattern for the Fc gamma receptor IIB (FcγRIIB) in a sample of the individual. The presence of the homozygous FcγRIIB 232 T/T genotype is found to be indicative of an individual that will exhibit a negative therapeutic response to said Fc gamma receptor IIB (FcγRIIB) therapy, whereas the presence of the homozygous FcγRIIB 232 I/I genotype is found to be indicative of an individual that will exhibit a positive therapeutic response to said Fc gamma receptor IIB (FcγRIIB) therapy.

The present invention also provides a method of treating an individual suffering from ITP with a Fc gamma receptor IIB (FcγRIIB) therapy where the individual is characterized by the homozygous FcγRIIB 232 I/I genotype. The method comprises administering a therapeutically effective amount of said Fc gamma receptor IIB (FcγRIIB) therapy to said individual.

In another aspect, also provided is a Fc gamma receptor IIB (FcγRIIB) therapy for use in a method of treating an individual suffering from ITP, wherein said individual is characterized by the homozygous FcγRIIB 232 I/I genotype, and wherein said method comprises administering an therapeutically effective amount of said Fc gamma receptor IIB (FcγRIIB) therapy to said individual.

The present invention provides a method of treating an individual suffering from ITP with a Fc gamma receptor IIB (FcγRIIB) therapy, comprising detecting the presence of the allelic pattern for the Fc gamma receptor IIB (FcγRIIB) in a sample of said individual, wherein the presence of the homozygous FcγRIIB 232 I/I genotype is indicative of an individual that will exhibit a positive therapeutic response to said Fc gamma receptor IIB (FcγRIIB) therapy.

Also provided is a Fc gamma receptor IIB (FcγRIIB) therapy, for use in a method of treating an individual suffering from ITP with a Fc gamma receptor IIB (FcγRIIB) therapy, said method comprising detecting the presence of the allelic pattern for the Fc gamma receptor IIB (FcγRIIB) in a sample of said individual, wherein the presence of the homozygous FcγRIIB 232 I/I genotype is indicative of an individual that will exhibit a positive therapeutic response to said Fc gamma receptor IIB (FcγRIIB) therapy.

In another aspect, the present invention provides a method for stratifying individual suffering from ITP with regard to their susceptibility to treatment with a Fc gamma receptor IIB (FcγRIIB) therapy, comprising determining the allelic pattern for the Fc gamma receptor IIB (FcγRIIB) in a sample of said individual, wherein the presence of the homozygous FcγRIIB 232 T/T genotype is indicative of an individual that will exhibit a negative therapeutic response to said Fc gamma receptor IIB (FcγRIIB) therapy.

Also provided is a method for stratifying individual suffering from ITP with regard to their susceptibility to treatment with a Fc gamma receptor IIB (FcγRIIB) therapy, comprising determining the allelic pattern for the Fc gamma receptor IIB (FcγRIIB) in a sample of said individual, wherein the presence of the homozygous FcγRIIB 232 I/I genotype is indicative of an individual that will exhibit a positive therapeutic response to said Fc gamma receptor IIB (FcγRIIB) therapy.

Furthermore, the present invention provides a method of treating an individual suffering from ITP and having the homozygous FcγRIIB 232 T/T genotype, said method comprising administering a therapeutically effective amount of an alternative ITP therapy such as Romiplostim, Intravenous immunoglobulin (IVIG), Revolade, and/or the antibodies disclosed in EP-B1 1 876 240 to said individual.

In another aspect, the present invention provides an alternative ITP therapy such as Romiplostim, Intravenous immunoglobulin (IVIG), Revolade, and/or the antibodies disclosed in EP-B1 1 876 240for use in a method of treating an individual suffering from ITP and having the homozygous FcγRIIB 232 T/T genotype, said method comprising administering an therapeutically effective amount of said Romiplostim therapy to said individual.

Provided here is also a pharmaceutical package comprising an Fc gamma receptor IIB (FcγRIIB) therapy The package may comprise (a) instructions and/or an imprint indicating that said Fc gamma receptor IIB (FcγRIIB) therapy is to be used for the treatment of an individual (i) suffering from ITP and (ii) being characterized by the homozygous FcγRIIB 232 I/I genotype; and/or (b) instructions and/or an imprint indicating that the individual to be treated shall be stratified by a method described in the present invention.

In the methods described here, the Fc gamma receptor IIB (FcγRIIB) therapy may be selected from the group consisting of a soluble Fc gamma receptor IIB as described herein.

### DETAILED DESCRIPTION OF THE PRESENT INVENTION

The present invention is based on the surprising discovery that an individual who is homozygous for the FcγRIIB^{T232} allele have less positive response to a FcγRIIB therapy than an individual who is not homozygous for the FcγRIIB^{T232}. In particular, an individual who is homozygous for the FcγRIIB^{I2-32} allele was discovered to respond positively to a FcγRIIB therapy.

This is useful to predicting how an individual is afflicted with a disease requiring FcγRIIB therapy would respond to the treatment.

Accordingly, in a first aspect the present invention relates to a method for predicting therapeutic response to Fc gamma receptor IIB (FcγRIIB) therapy in an individual suffering from Idiopathic thrombocytopenic purpura (ITP), comprising detecting the presence of the allelic pattern for the Fc gamma receptor IIB (FcγRIIB) in a sample of said individual, wherein the presence of the homozygous FcγRIIB 232 T/T genotype is indicative of an individual that will exhibit a negative therapeutic response to said Fc gamma receptor IIB (FcγRIIB) therapy.

The present invention is also related to a method for predicting therapeutic response to Fc gamma receptor IIB (FcγRIIB) therapy in an individual suffering from idiopathic thrombocytopenic purpura (ITP), comprising detecting the presence of the allelic pattern for the Fc gamma receptor IIB (FcγRIIB) in a sample of said individual, wherein the presence of the homozygous FcγRIIB 232 I/I genotype is indicative of an individual that will exhibit a positive therapeutic response to said Fc gamma receptor IIB (FcγRIIB) therapy
"Predicting" or "prediction" of a therapeutic response, within the meaning of the invention, shall be understood to be the act of determining a likely outcome of a FcγRIIB therapy in an individual or patient. The prediction of a response is preferably made with reference to probability values for reaching a desired or non-desired outcome of the chemotherapy. The predictive methods of the present invention can be used clinically to make treatment decisions by choosing the most appropriate treatment modalities for any particular patient.

"FcyRIIB therapy" shall be understood as the treatment of an individual in need thereof with a soluble FcγRIIB receptor, romiplastin (Nplate®), eltrombopag (Promacta®), and/or IVIG (see Anthony et al. (2008), Proc Natl Acad Sci U S A. 105(50):19571-19578).

According to the present invention, the preparation of the soluble FcγRIIB receptor takes place in prokaryotic or eukaryotic cells. It may also take place in eukaryotic cells. If it takes place in prokaryotic cells (see EP-B1 1 135 486) insoluble inclusion bodies containing the recombinant protein form, thus facilitating purification by separation of the inclusion bodies from other cell components before renaturation of the proteins contained therein takes place. The renaturation of the soluble FcγRIIB receptor according to the present invention which are contained in the inclusion bodies can principally take place according to known methods. The advantage of the preparation in prokaryotic cells, the production of inclusion bodies and the thus obtained recombinant soluble FcγRIIB receptors make it possible to obtain a very pure and, in particular, also very homogeneous FcR preparation. Also because of the absence of glycosylation the obtained product is of great homogeneity. However, in some cases glycosylation may be desired.

A host cell is genetically engineered with the polynucleotide or the vector encoding or carrying the soluble FcγRIIB receptor. The host cells that may be used for purposes of the invention include but are not limited to prokaryotic cells such as bacteria (for example, E. coli and B. subtilis), which can be transformed with, for example, recombinant bacteriophage DNA, plasmid DNA, or cosmid DNA expression vectors containing the polynucleotide molecules encoding the FcR; simple eukaryotic cells like yeast (for example, Saccharomyces and Pichia), which can be transformed with, for example, recombinant yeast expression vectors containing the polynucleotide molecule of the invention, i.e. the polynucleotide molecules encoding the FcR; insect cell systems like, for example, Sf9 of Hi5 cells, which can be infected with, for example, recombinant virus expression vectors (for example, baculovirus) containing the polynucleotide molecules of the invention; Xenopus oocytes, which can be injected with, for example, plasmids; plant cell systems, which can be infected with, for example, recombinant virus expression vectors (for example, cauliflower mosaic virus (CaMV) or tobacco mosaic virus (TMV)) or transformed with recombinant plasmid expression vectors (for example, Ti plasmid) containing a FcR or variant nucleotide sequence; or mammalian cell systems (for example, COS, CHO, BHK, HEK293, VERO, HeLa, MDCK, Wi38, Swiss 3T3 and NIH 3T3 cells), which can be transformed with recombinant expression constructs containing, for example, promoters derived, for example, from the genome of mammalian cells (for example, the metallothionein promoter) from mammalian viruses (for example, the adenovirus late promoter, CMV IE and the vaccinia virus 7.5K promoter) or from bacterial cells (for example, the tet-repressor binding is employed in the tet-on and tet-off systems). Also useful as host cells are primary or secondary cells obtained directly from a mammal and transfected with a plasmid vector or infected with a viral vector. Depending on the host cell and the respective vector used to introduce the polynucleotide of the invention the polynucleotide can integrate, for example, into the chromosome or the mitochondrial DNA or can be maintained extrachromosomally like, for example, episomally or can be only transiently comprised in the cells.

In the sequence of FcyRllb three potential N-glycosylation sites are found. All three sites are on the surface of the molecule and are accessible. They are located in the EIF loops (N61 and N142) of both domains and on strand E (N 135) of the C-terminal domain. FcRs isolated from mammalian cells are highly glycosylated. Since FcR is glycosylated in vivo it may be desirable to choose an expression system, which provides faithful glycosylation of the protein. Consequently, it is preferred to introduce the polynucleotides encoding the FcR of the present invention into higher eukaryotic cells, in particular into mammalian cells, e.g. COS, CHO, BHK, HEK293, VERO, HeLa, MDCK, Wi38, Swiss 3T3 or NIH 3T3 cells.

Preferably the FcγRIIB receptor according to the invention lacks the transmembrane domain and/or the signal peptide and is soluble. It is also envisaged that the FcγRIIB receptor according to the invention is of human origin.

In a particularly preferred embodiment, said FcγRIIB receptor is a protein which is encoded by SEQ ID No. 2 or 4 (SEQ ID No 4 being most preferred), and/or a protein which comprises, contains or is identical with the amino acid sequence depicted in SEQ ID Nos. 1 or 3 (SEQ ID No 3 being most preferred).
SEQ ID No. 1 
MGTPAAPPKA VLKLEPQWIN VLQEDSVTLT CRGTHSPESD SIQWFHNGNL IPTHTQPSYR
FKANNNDSGE YTCQTGQTSL SDPVHLTVLS EWLVLQTPHL EFQEGETIVL RCHSWKDKPL
VKVTFFQNGK SKKFSRSDPN FSIPQANHSH SGDYHCTGNI GYTLYSSKPV TITVQAPSSS
P
SEQ ID No. 2 
1 atggggacac ctgcagctcc cccaaaggct gtgctgaaac tcgagcccca gtggatcaac
61 gtgctccagg aggactctgt gactctgaca tgccggggga ctcacagccc tgagagcgac
121 tccattcagt ggttccacaa tgggaatctc attcccaccc acacgcagcc cagctacagg
181 ttcaaggcca acaacaatga cagcggggag tacacgtgcc agactggcca gaccagcctc
241 agcgaccctg tgcatctgac tgtgctttct gagtggctgg tgctccagac ccctcacctg
301 gagttccagg agggagaaac catcgtgctg aggtgccaca gctggaagga caagcctctg
361 gtcaaggtca cattcttcca gaatggaaaa tccaagaaat tttcccgttc ggatcccaac
421 ttctccatcc cacaagcaaa ccacagtcac agtggtgatt accactgcac aggaaacata
481 ggctacacgc tgtactcatc caagcctgtg accatcactg tccaagctcc cagctcttca
541 ccg
SEQ ID No. 3 
MAPPKAVLKL EPQWINVLQE DSVTLTCRGT HSPESDSIQW FHNGNLIPTH
TQPSYRFKAN NNDSGEYTCQ TGQTSLSDPV HLTVLSEWLV LQTPHLEFQE
GETIVLRCHS WKDKPLVKVT FFQNGKSKKF SRSDPNFSIP QANHSHSGDY
HCTGNIGYTL YSSKPVTITV QAPSSSP
SEQ ID No. 4

1 ATGGCACCGC CGAAAGCAGT TCTGAAACTG GAACCGCAGT GGATTAACGT TCTGCAGGAA
61 GATAGCGTTA CCCTGACCTG TCGTGGCACC CATAGCCCGG AAAGCGATAG CATTCAGTGG
121 TTTCACAACG GCAATCTGAT TCCGACCCAT ACCCAGCCGA GCTATCGTTT TAAAGCGAAC
181 AACAACGATA GCGGCGAATA TACCTGTCAG ACCGGTCAGA CCAGCCTGAG CGATCCGGTT
241 CATCTGACCG TTCTGAGCGA ATGGCTGGTT CTGCAGACCC CGCATCTGGA ATTTCAGGAA
301 GGCGAAACCA TTGTTCTGCG TTGCCACAGC TGGAAAGATA AACCGCTGGT TAAAGTTACC
361 TTCTTCCAGA ACGGCAAAAG CAAAAAATTC AGCCGTAGCG ATCCGAATTT TAGCATTCCG
421 CAGGCGAATC ATAGCCATAG CGGCGATTAT CATTGTACCG GCAACATTGG CTATACCCTG
481 TATAGCAGCA AACCGGTGAC CATTACCGTT CAGGCGCCGA GCAGCAGCCC GTAA

The FcγRIIB receptor polypeptides can be any of those described herein but with not more than ten (e.g., not more than: ten, nine, eight, seven, six, five, four, three, two, or one) conservative substitutions. Conservative substitutions are known in the art and typically include substitution of, e.g. one polar amino acid with another polar amino acid and one acidic amino acid with another acidic amino acid. Accordingly, conservative substitutions preferably include substitutions within the following groups of amino acids: glycine, alanine, valine, proline, isoleucine, and leucine (non polar, aliphatic side chain); aspartic acid and glutamic acid (negatively charged side chain); asparagine, glutamine, methionine, cysteine, serine and threonine (polar uncharged side chain); lysine, histidine and arginine; and phenylalanine, tryptophane and tyrosine (aromatic side chain); and lysine, arginine an histidine (positively charged side chain). It is well known in the art how to determine the effect of a given substitution, e.g. on pKI etc. All that is required of a polypeptide having one or more conservative substitutions is that it has at least 50% (e.g., at least: 55%; 60%; 65%, 70%; 75%; 80%; 85%; 90%; 95%; 98%; 99%; 99.5%; or 100% or more) of the activity of the unaltered Fc[gamma] receptor according to the invention.

Both polypeptides and peptides can be produced by standard in vitro recombinant DNA techniques and in vivo transgenesis, using nucleotide sequences encoding the appropriate polypeptides or peptides. Methods well-known to those skilled in the art can be used to construct expression vectors containing relevant coding sequences and appropriate transcriptional/translational control signals. See, for example, the techniques described in Sambrook, et al., Molecular Cloning: A Laboratory Manual (2nd Ed.) [Cold Spring Harbor Laboratory, N.Y., 1989], and Ausubel, et al., Current Protocols in Molecular Biology [Green Publishing Associates and Wiley Interscience, N.Y., 1989].

The FcγRIIB receptor according to the invention may be chemically modified (improved) by PEGylation and/or genetic engineering.

Known approaches involve the provision of additional glycosylation sites (see e.g. WO 91/05867 , WO 94/09257 and WO 01/81405). Such modified analogs may have at least one additional N-linked and/or O-linked carbohydrate chain. Other attempts to improve the half-life may involve the addition of polyethylene glycol residues (PEG) of varying length to the amino acid backbone (see e.g. WO 00/32772, WO 01/02017, WO 03/029291). One may modify the molecules with at least one N-linked and/or O-linked oligosaccharide which is further modified by oxidation, sulfation, phosphorylation PEGylation or a combination thereof (see WO 2005/025606). All these approaches can equally be employed to extend the half-life of the variants of the present invention and accordingly in a preferred embodiment of the Fc[gamma] receptor according to the invention the modification is selected from the group consisting of oxidation, sulfation, phosphorylation, addition of oligosaccharides or combinations thereof. If the addition of further N-linked or O-linked oligonucleotides is desired it is possible to introduce them by introducing additional glycosylation sites. It also preferred that the protein is affinity modulated.

In the practice of one aspect of the present invention, a pharmaceutical composition comprising the soluble FcγRIIB receptor of the invention may be administered to a mammal by any route which provides a sufficient level of activity. It can be administered systemically or locally. Such administration may be parenterally, transmucosally, e.g., orally, nasally, rectally, intravaginally, sub-lingually, submucosally or transdermally. Preferably, administration is parenteral, e.g., via intravenous or intraperitoneal injection, and also including, but is not limited to, intraarterial, intramuscular, intradermal and subcutaneous administration. If the pharmaceutical composition of the present invention is administered locally it can be injected directly into the organ or tissue to be treated. In cases of treating the nervous system this administration route includes, but is not limited to, the intracerebral, intraventricular, intracerebroventricular, intrathecal, intracistemal, intraspinal and/or peri-spinal routes of administration, which can employ intracranial and intravertebral needles, and catheters with or without pump devices.

The prediction of a response is preferably made with reference to probability values for reaching a desired or non-desired outcome of the therapy. The predictive methods of the present invention can be used clinically to make treatment decisions by choosing the most appropriate treatment modalities for any particular patient. In the context of the present invention, a "positive response" or "positive therapeutic response" to a medicament or agent can be defined as comprising a reduction of the symptoms related to ITP. Such symptoms may be one or more of the following: easy or excessive bruising (purpura); superficial bleeding (petechiae); prolonged bleeding from cuts; spontaneous bleeding from gums or nose, blood in urine or stools; menorrhagia; profuse bleeding during surgery; low platelet count (such as below 20,000 per µl); internal bleeding, subarachnoid or intracerebral hemorrhage; lower gastrointestinal bleeding or other known symptoms. In the context of the present invention, a "negative response" to the therapy is defined as lack of positive response to the medicament or the development of adverse side-effects.

"Treat" or "treatment" as used herein, means to reduce, stabilize, or inhibit progression of a symptom of ITP.

### FcγRIIB

FcγRIIB is one of the receptors for the Fc region of IgG (FcγRs). FcγRs are expressed by many immune cells and are important in both promoting and regulating the immune and inflammatory response to immune complexes. Most FcγRs are activating receptors and include the high-affinity receptor FcγRI and a family of low affinity receptors, including FcγRIIA, FcγRIIC, FcγRIIIA and FcγRIIIB in humans. FcγRIIB is the only FcγR that has an inhibitory function.

FcγRIIB has an important role in controlling humoral immunity by regulating B cell activation, localization of B cells in the germinal centers, as well as plasma cell survival. FcγRIIB regulates B cell activation by increasing the B cell receptor activation threshold and suppressing B cell-mediated antigen presentation to T cells. FcγRIIB also influences antigen presentation by inhibiting FcγR-dependent internalization of immune-complexed antigen by DCs, as well antigen presentation to both CD4+ and CD8+ T cells. FcγRIIB additionally inhibits IgE-induced mast cell and basophil degranulation, thus contributing to hypersensitivity responses. FcγRIIB also inhibits IgE-induced mast cell and basophil degranulation, thus contributing to hypersensitivity responses.

### Genetic variation of FcγRIIB

The FcγRII and FcγRIII receptor families is encoded by five FCGR genes (FCGR2A, FCGR2B, FCGR2C, FCGR3A and FCGR3B) located on the human 1q23.3 locus. Genetic variation at the FcR locus had been found to exist within different individuals. Several single nucleotide polymorphisms (SNPs) have been identified within the promoter and coding regions of FCGR2B. Among one of them, a non-synonymous T-to-C transition (rs1050501) in exon 5 of the gene, results in the substitution of a threonine (T) for isoleucine (I) at position 232 within the transmembrane domain of FcγRIIB (FcγRIIB^{T232}).

For the purpose of the present invention, FcγRIIB 232 I/I genotype means when an individual who are homozygous for FcγRIIB^{I232}. FcγRIIB 232 T/T genotype means when an individual who are homozygous for FcγRIIB^{T232}.

The determination of therapeutic response to Fc gamma receptor IIB includes detecting the presence of the homozygous FcγRIIB 232 I/I genotype or a homozygous FcγRIIB 232 T/T genotype in the patient. If the homozygous T/T genotype is detected, the individual will exhibit a negative therapeutic response to Fc gamma receptor IIB therapy; if the homozygous I/I genotype is detected, the individual will exhibit a positive therapeutic response to the therapy.

The term "genotype" as used herein refers the identity of the alleles present in an individual or a sample. In the context of the present invention a genotype preferably refers to the description of the alleles present in an individual or a sample. The term "genotyping" a sample or an individual for an allele involves determining the specific allele carried by an individual.

The term "allele" is used herein to refer to a variant of a nucleotide sequence. For example, alleles of the GHR nucleotide sequence coding regions of FCGR2B.

The term "232" when used herein in the context of a genotype or allelic pattern refers to the amino acid position 232 of the amino acid sequence of FcγRIIB which is, for example, depicted in Figure 2 of Brooks et al. (1989), J. Exp. Med. 170: 1369-1385. The amino acid sequence shown in said Figure 2 is the preferred reference sequence of FcγRIIB that is referred to in the methods and uses of the present invention. Accordingly, the reference sequence is applied when determining amino acid position 232 within a FcγRIIB amino acid sequence, i.e., when determining as to whether an amino acid within a FcγRIIB corresponds to position 232 of the FcγRIIB reference sequence of the invention. For example, in order to determine whether an amino acid residue in a given FcγRIIB sequence corresponds to a certain position in the amino acid of Figure 2 of Brooks et al. (cited above), the skilled person can use means and methods well-known in the art, e.g., alignments, either manually or by using computer programs such as those mentioned further down below in connection with the definition of the term "hybridization" and degrees of homology.

For example, BLAST2.0, which stands for Basic Local Alignment Search Tool (Altschul, Nucl. Acids Res. 25 (1997), 3389-3402; Altschul, J. Mol. Evol. 36 (1993), 290-300; Altschul, J. Mol. Biol. 215 (1990), 403-410), can be used to search for local sequence alignments. Another example for a program capable of generating sequence alignments is the CLUSTALW computer program (Thompson, Nucl. Acids Res. 2 (1994), 4673-4680) or FASTDB (Brutlag Comp. App. Biosci. 6 (1990), 237-245), as known in the art.

The term "position" when used in accordance with the present invention means the position of an amino acid within a FcγRIIB amino acid sequence. The term "corresponding" as used herein also includes that a position is not only determined by the number of the amino acids. For example, the position of a given amino acid sequence in accordance with the present invention may vary, for example, due to deletions or insertions (for example, because of isoforms, truncated forms, etc.) elsewhere in a FcγRIIB amino acid sequence. Thus, under a "corresponding position" in accordance with the present invention it is to be understood that amino acids may differ in the indicated number but may still have similar neighboring amino acids.

It is, however, possible for the skilled person to identify the amino acid at position 232 in the amino acid sequence of a FcγRIIB amino acid sequence and to also determine as to whether it corresponds to the FcγRIIB amino acid sequence shown in Figure 2 of Brooks et al. (cited above) by, for example, aligning the amino acid sequences as described herein.

The skilled person is aware that for determining the genotype or allelic pattern of FcγRIIB of an individual, it is advantageous to perform the analysis on nucleotide sequence level, though the analysis could also be done on amino acid level, e.g., by protein sequencing, MALDI-TOF, etc. or by way of an antibody that is capable of distinguishing different amino acids, in particular isoleucine from threonine, or *vice versa* at the position 232 of FcγRIIB. The skilled person knows that the amino acid isoleucine is encoded by the following codons: AUU, AUC or AUA, while the amino acid threonine is encoded by the following codons: ACU, ACC, ACA or ACG. Thus, a change from U to C at the second position of any of the codons that encoe isoleucine results in the amino acid threonine. A change in the second position from U to C and a change in the third position from U, C or A to G also results in threonine. Accordingly, the skilled person can easily determine the codon encoding the amino acid sequence at position 232 of FcγRIIB encoding nucleotide sequence by means and methods known in the art, or those described exemplarily herein.

### Method of determining allelic pattern

The methods disclosed in the present application include detecting the presence of the allelic pattern for the FcγRIIB in a individual. This is done *in vitro* by first obtaining a sample from the individual. The sample may be obtained prior to, during, or after the treatment. In accordance with the present invention by the term "sample" is intended any biological sample obtained from a subject, cell line, tissue culture, or other source containing genomic DNA of the individual. Biological samples include body fluids (such as blood, serum, plasma, urine, saliva, synovial fluid and spinal fluid) and tissue sources found to contain B cell, T cell, dendritic cells, macrophages, neutrophils, and mast cells. Methods for obtaining tissue biopsies and body fluids from subjects are well known in the art.

For example, identification of the allelic variant can be carried out using an OLA (U.S. Pat. No. 4,998,617; Landegren et al., Science 241: 1077, 1988). The OLA protocol uses two oligonucleotides, which are designed to be capable of hybridizing to abutting sequences of a single strand of a target. One of the oligonucleotides is linked to a separation marker, e.g., biotinylated, and the other is detectably labeled. If the precise complementary sequence is found in a target molecule, the oligonucleotides will hybridize such that their termini abut, and create a ligation substrate. Ligation then permits the labeled oligonucleotide to be recovered using avidin, or another biotin ligand. Nickerson et al., have described a nucleic acid detection assay that combines attributes of PCR and OLA (Nickerson et al., PNAS 87: 8923-8927, 1990). In this method, PCR is used to achieve the exponential amplification of target DNA, which is then detected using OLA. For example, U.S. Pat. No. 5,593,826 discloses an OLA using an oligonucleotide having 3'-amino group and a 5'-phosphorylated oligonucleotide to form a conjugate having a phosphoramidate linkage. In another variation, OLA combined with PCR permits typing of two alleles in a single microtiter well (Tobe et al., Nucleic Acids Res. 24: 3728, 1996). By marking each of the allele-specific primers with a unique hapten, e.g., digoxigenin and fluorescein, each OLA reaction can be detected by using hapten specific antibodies that are labeled with different enzyme reporters, alkaline phosphatase, or horseradish peroxidase. This system permits the detection of the two alleles using a high throughput format that leads to the production of two different colors. In other embodiments, an SNP can be detected by using a specialized exonuclease-resistant nucleotide (see, for example, U.S. Pat. No. 4,656,127, the contents of which are hereby incorporated by reference). According to the method, a primer complementary to the allelic sequence immediately 3' to the polymorphic site is permitted to hybridize to the target molecule obtained from the subject or reference control cells. If the polymorphic site on the target molecule contains a nucleotide that is complementary to the particular exonuclease-resistant nucleotide derivative present, then that derivative will be incorporated onto the end of the hybridized primer. Such incorporation renders the primer resistant to exonuclease, and thereby permits its detection. Since the identification of the exonuclease-resistant derivative of the sample is known, a finding that the primer has become resistant to exonucleases reveals that the nucleotide present in the polymorphic site of the target molecule was complementary to that of the nucleotide derivative used in the reaction. This method has the advantage that it does not require the determination of large amount of extraneous sequence data. In another embodiment, a solution-based method is used for determining the identity of the nucleotide of a polymorphic site (International Publication No. WO 91/02087). A primer is employed that is complementary to allelic sequences immediately 3' to a polymorphic site. The method determines the identity of the nucleotide of that site using labeled dideoxynucleotide derivatives, which, if complementary to the nucleotide of the polymorphic site will become incorporated onto the terminus of the primer.

In still a further embodiment, a method known as Genetic Bit Analysis (GBA™) can be used to determine genotype (International Publication No. WO 92/15712). The method uses mixtures of labeled terminators and primer that is complementary to the sequence 3' to a polymorphic site. The labeled terminator that is incorporated is thus determined by, and complementary to, the nucleotide present in the polymorphic site of the target molecule being evaluated.

A skilled person is able to design suitable PCR primers for amplifying this region in view of the teaching of Kyogoku, C. et al. Arthritis Rheum. 46, 1242-1254 (2002); Willcocks et al. Proc. Natl Acad. Sci. USA. 107(17):7881-5 2010; Kono et al. Hum Mol Genet. 2005 Oct 1;14(19):2881-92 (2005); Xu et al. Platelets, 2010; 21(6): 479-485; Duan et al Scand J Rheumatol. 2012; Pan et al Lupus. 2008 17(8):733-8.

"Primer" denotes a specific oligonucleotide sequence which is complementary to a target nucleotide sequence and used to hybridize to the target nucleotide sequence. A primer serves as an initiation point for nucleotide polymerization catalyzed by either DNA polymerase, RNA polymerase or reverse transcriptase. Thus, the term primer, as defined herein, is meant to encompass any nucleic acid that is capable of priming the synthesis of a nascent nucleic acid in a template-dependent process. Typically, primers are oligonucleotides from ten to twenty base pairs in length, but longer sequences can be employed.

The methods of the invention will also be useful in assessing and conducting clinical trials of a FcγRIIB therapy. The methods accordingly comprise identifying a first population of individuals who respond positively to the medicament and a second population of individuals who respond negatively to the medicament or whose positive response to said medicament is diminished in comparison to said first population of individuals. In one embodiments, the medicament may be administered to the subject in a clinical trial if the DNA sample suggests that the subject homozygous FcγRIIB 232 I/I genotype.

The DNA sample obtained from the individual may be first amplified. A number of template dependent processes are available to amplify the marker sequences present in a given template sample. One of the best known amplification methods is the polymerase chain reaction (referred to as PCR) which is described in detail in U.S. Pat. No. Nos. 4,683,195, 4,683,202 and 4,800,159, and in Innis et al., PCR Protocols, Academic Press, Inc. San Diego Calif., 1990., each of which is incorporated herein by reference in its entirety.

Briefly, in PCR, two primer sequences are prepared that are complementary to regions on opposite complementary strands of the marker sequence. An excess of deoxynucleoside triphosphates are added to a reaction mixture along with a DNA polymerase, e.g., Taq polymerase. If the marker sequence is present in a sample, the primers will bind to the marker and the polymerase will cause the primers to be extended along the marker sequence by adding on nucleotides. By raising and lowering the temperature of the reaction mixture, the extended primers will dissociate from the marker to form reaction products, excess primers will bind to the marker and to the reaction products and the process is repeated.

A reverse transcriptase PCR amplification procedure may be performed in order to quantify the amount of mRNA amplified. Methods of reverse transcribing RNA into cDNA are well known and described in Sambrook et al., In: Molecular Cloning. A Laboratory Manual. 2d Ed., Cold Spring Harbor Laboratory Press, Cold Spring Harbor, N.Y., 1989. Alternative methods for reverse transcription utilize thermostable, RNA-dependent DNA polymerases. These methods are described in WO 90/07641. Polymerase chain reaction methodologies are well known in the art. Another method for amplification is the ligase chain reaction ("LCR" U.S. Pat. Nos. 5,494,810, 5,484,699, EPO No. 320 308, each incorporated herein by reference). In LCR, two complementary probe pairs are prepared, and in the presence of the target sequence, each pair will bind to opposite complementary strands of the target such that they abut. In the presence of a ligase, the two probe pairs will link to form a single unit.

By temperature cycling, as in PCR, bound ligated units dissociate from the target and then serve as "target sequences" for ligation of excess probe pairs. U.S. Pat. No. 4,883,750 describes a method similar to LCR for binding probe pairs to a target sequence.

Qbeta-Replicase, an RNA-directed RNA polymerase, can be used as yet another amplification method in the present invention. In this method, a replicative sequence of RNA that has a region complementary to that of a target is added to a sample in the presence of an RNA polymerase. The polymerase will copy the replicative sequence that can then be detected. Similar methods also are described in U.S. Pat. No. 4,786,600, incorporated herein by reference, which concerns recombinant RNA molecules capable of serving as a template for the synthesis of complementary single-stranded molecules by RNA-directed RNA polymerase. The product molecules so formed also are capable of serving as a template for the synthesis of additional copies of the original recombinant RNA molecule.

An isothermal amplification method, in which restriction endonucleases and ligases are used to achieve the amplification of target molecules that contain nucleotide 5'-[alpha-thio]- triphosphates in one strand of a restriction site also may be useful in the amplification of nucleic acids in the present invention (Walker et al, (1992), Proc. Nat'l Acad Sci. USA, 89:392-396; U.S. Pat. No. 5,270,184 incorporated herein by reference). U.S. Pat. No. 5,747,255 (incorporated herein by reference) describes an isothermal amplification using cleavable oligonucleotides for polynucleotide detection. In the method described therein, separated populations of oligonucleotides are provided that contain complementary sequences to one another and that contain at least one scissile linkage which is cleaved whenever a perfectly matched duplex is formed containing the linkage. When a target polynucleotide contacts a first oligonucleotide cleavage occurs and a first fragment is produced which can hybridize with a second oligonucleotide. Upon such hybridization, the second oligonucleotide is cleaved releasing a second fragment that can in turn, hybridize with a first oligonucleotide in a manner similar to that of the target polynucleotide.

Strand Displacement Amplification (SDA) is another method of carrying out isothermal amplification of nucleic acids which involves multiple rounds of strand displacement and synthesis, i.e., nick translation (e.g., U.S. Pat. Nos. 5,744,311; 5,733,752; 5,733,733; 5,712,124). A similar method, called Repair Chain Reaction (RCR), involves annealing several probes throughout a region targeted for amplification, followed by a repair reaction in which only two of the four bases are present. The other two bases can be added as biotinylated derivatives for easy detection. A similar approach is used in SDA. Target specific sequences can also be detected using a cyclic probe reaction (CPR). In CPR, a probe having 3' and 5' sequences of non-specific DNA and a middle sequence of specific RNA is hybridized to DNA that is present in a sample. Upon hybridization, the reaction is treated with RNase H, and the products of the probe identified as distinctive products that are released after digestion. The original template is annealed to another cycling probe and the reaction is repeated.

Still another amplification methods described in GB Application No. 2 202 328, and in PCT Application No. PCT/US89/01025, each of which is incorporated herein by reference in its entirety, may be used in accordance with the present invention. In the former application, "modified" primers are used in a PCR-like, template-and enzyme-dependent synthesis. The primers may be modified by labeling with a capture moiety (e.g., biotin) and/or a detector moiety (e.g., enzyme). In the latter application, an excess of labeled probes are added to a sample. In the presence of the target sequence, the probe binds and is cleaved catalytically. After cleavage, the target sequence is released intact to be bound by excess probe. Cleavage of the labeled probe signals the presence of the target sequence.

Other nucleic acid amplification procedures include transcription-based amplification systems (TAS), including nucleic acid sequence based amplification (NASBA) and 3SR (Kwok et al., (1989) Proc. Nat'l Acad. Sci. USA, 86: 1173; and WO 88/10315, incorporated herein by reference in their entirety). In NASBA, the nucleic acids can be prepared for amplification by standard phenol/chloroform extraction, heat denaturation of a clinical sample, treatment with lysis buffer and minispin columns for isolation of DNA and RNA or guanidinium chloride extraction of RNA. These amplification techniques involve annealing a primer which has target specific sequences. Following polymerization, DNA/RNA hybrids are digested with RNase H while double stranded DNA molecules are heat denatured again. In either case the single stranded DNA is made fully double stranded by addition of second target specific primer, followed by polymerization. The double-stranded DNA molecules are then multiply transcribed by an RNA polymerase such as T7 or SP6. In an isothermal cyclic reaction, the RNA's are reverse transcribed into single stranded DNA, which is then converted to double stranded DNA, and then transcribed once again with an RNA polymerase such as T7 or SP6. The resulting products, whether truncated or complete, indicate target specific sequences.

Davey et al., EPO No. 329 822 (incorporated herein by reference in its entirety) disclose a nucleic acid amplification process involving cyclically synthesizing single-stranded RNA ("ssRNA"), ssDNA; and double-stranded DNA (dsDNA), which may be used in accordance with the present invention. The ssRNA is a template for a first primer oligonucleotide, which is elongated by reverse transcriptase (RNA-dependent DNA polymerase). The RNA is then removed from the resulting DNA:RNA duplex by the action of ribonuclease H (RNase H, an RNase specific for RNA in duplex with either DNA or RNA). The resultant ssDNA is a template for a second primer, which also includes the sequences of an RNA polymerase promoter (exemplified by T7 RNA polymerase) 5' to its homology to the template. This primer is then extended by DNA polymerase (exemplified by the large "Klenow" fragment of E. coli DNA polymerase I), resulting in a double-stranded DNA ("dsDNA") molecule, having a sequence identical to that of the original RNA between the primers and having additionally, at one end, a promoter sequence. This promoter sequence can be used by the appropriate RNA polymerase to make many RNA copies of the DNA. These copies can then re-enter the cycle leading to very swift amplification. With proper choice of enzymes, this amplification can be done isothermally without addition of enzymes at each cycle. Because of the cyclical nature of this process, the starting sequence can be chosen to be in the form of either DNA or RNA.

PCT Application WO 89/06700 (incorporated herein by reference in its entirety) disclose a nucleic acid sequence amplification scheme based on the hybridization of a promoter/primer sequence to a target single-stranded DNA ("ssDNA") followed by transcription of many RNA copies of the sequence. This scheme is not cyclic, i.e., new templates are not produced from the resultant RNA transcripts. Other amplification methods include "RACE" and "one-sided PCR" (Frohman,, In: PCR Protocols. A Guide To Methods And Applications, Academic Press, N.Y., 1990; and O'hara et al., (1989) Proc. Nat'l Acad. Sci. USA, 86: 5673-5677; each herein incorporated by reference in their entireties).

Amplified nucleic acids can be assayed to determine the genotype by any of a variety of methods, including but not limited to allele-specific oligonucleotide (ASO) probing, differential restriction endonuclease digestion, ligase-mediated gene detection (LMGD), gel electrophoresis, oligonucleotide ligation assay (OLA), exonuclease-resistant nucleotides, and genetic bit analysis (GBA). Additional methods of analysis can also be useful, such as fluorescence resonance energy transfer (FRET) (Wolf et. al., PNAS 85: 8790-94, 1988). SSCP (single strand confirmation polymorphism) and DHPLC (denaturing high-performance liquid chromatography) can also be employed. Any of these or other known methods can be employed to determine the presence or absence of polymorphic alleles. The methods employed or compositions used are not intended to be limited to any one polymorphism and should be construed to encompass all polymorphisms described herein.

The present invention is based on the surprising finding that the therapeutic response is highly related to the FcγRIIB genotype in the individual.

Provided herein is a method of treating an individual suffering from ITP with a Fc gamma receptor IIB (FcγRIIB) therapy. The method may optionally require the detecting the presence of the allelic pattern for the Fc gamma receptor IIB (FcγRIIB) in a sample of said individual. After having determined that the individual is characterized by the homozygous FcγRIIB 232 I/I genotype, a therapeutically effective amount of the Fc gamma receptor IIB (FcγRIIB) therapy is administered to said individual. As discussed, individuals who have FcγRIIB 232 I/I genotype are expected to have a greater positive response to the treatment than subjects that do not. In preferred aspects, a dose administered to subjects FcγRIIB 232 I/I genotype will be greater than the dose administered to a subject that do not have the genotype, such as FcγRIIB 232 T/T or FcγRIIB 232 T/I genotype.

In another aspect, the present invention provides Fc gamma receptor IIB (FcγRIIB) therapy for use in a method of treating an individual suffering from ITP and characterized by the homozygous FcγRIIB 232 I/I genotype. The therapy comprises administering a therapeutically effective amount of said Fc gamma receptor IIB (FcγRIIB) therapy agent to said individual. The therapy may be preceded by detecting the presence of the allelic pattern for the Fc gamma receptor IIB (FcγRIIB) in a sample, such as DNA sample of said individual. As stated earlier, the presence of the homozygous FcγRIIB 232 I/I genotype is indicative of an individual that will exhibit a positive therapeutic response to said Fc gamma receptor IIB (FcγRIIB) therapy.

The terms "subject", "individual", and "patient" are used interchangeably. They include mammalian and non-mammalian subjects; "mammal" for purposes of treatment refers to any animal classified as a mammal, including human, domestic and farm animals, nonhuman primates, and any other animal that has mammary tissue. A mammal includes human, rodents such as mouse, rat or rabbit, dog, cat, chimpanzee, horse, pig, etc., with human being preferred. A subject also includes human and veterinary patients, with human patients being preferred.

"Treat" or "treatment" as used herein, means to reduce, stabilize, or inhibit progression of a symptom, such as cancer size, number of metastases or other symptoms which are caused by/associated with the presence and/or progression of a cancer.

In accordance with the present invention by the term "sample" is intended any biological sample containing DNA obtained from a subject cell line, tissue culture, or other source containing autoantibodies, polynucleotides or polypeptides or portions thereof. Biological samples include body fluids (such as blood, serum, plasma, urine, saliva, synovial fluid and spinal fluid). Methods for obtaining tissue biopsies and body fluids from subjects are well known in the art. Generally, a biological sample which includes proteins, in particular antigens, autoantibodies and polynucleotides is preferred as a source. Other preferred samples are whole blood, serum, plasma or synovial fluid, with plasma or serum being most preferred.

The present invention also provides a method for stratifying individuals suffering from ITP with regard to their susceptibility to treatment with a Fc gamma receptor IIB (FcγRIIB) therapy, comprising determining the allelic pattern for the Fc gamma receptor IIB (FcγRIIB) in a sample of said individual, wherein the presence of the homozygous FcγRIIB 232 T/T genotype is indicative of an individual that will exhibit a negative therapeutic response to said Fc gamma receptor IIB (FcγRIIB) therapy.

In another aspect, the present invention provides a method for stratifying individuals suffering from ITP with regard to their susceptibility to treatment with a Fc gamma receptor IIB (FcγRIIB) therapy, comprising determining the allelic pattern for the Fc gamma receptor IIB (FcγRIIB) in a sample of said individual, wherein the presence of the homozygous FcγRIIB 232 I/I genotype is indicative of an individual that will exhibit a positive therapeutic response to said Fc gamma receptor IIB (FcγRIIB) therapy.

The term "stratify" or "stratifying" refers to sorting individuals or patients into those who are more likely to benefit from the Fc gamma receptor IIB (FcγRIIB) therapy from those who are less likely to benefit from the therapy. The methods of present invention may thus be employed for ITP cancer patients with regard to their susceptibility to treatment with Fc gamma receptor IIB (FcγRIIB) therapy. As mentioned, those patients who have homozygous FcγRIIB 232 I/I genotype are more likely to benefit from the therapy than those who do not.

Specifically, a "patient who may benefit" from Fc gamma receptor IIB (FcγRIIB) therapy is a patient in which the Fc gamma receptor IIB (FcγRIIB) therapy has a higher likelihood to have a therapeutic effect. The likelihood that (a) cancer and/or a cancer patient may or may not respond favorably is dependent on the symptoms observed on the patient.

The present invention also provides a method of treating an individual suffering from ITP and having the homozygous FcγRIIB 232 T/T genotype, said method comprising administering a therapeutically effective amount of an alternative ITP therapy such as Romiplostim, Intravenous immunoglobulin (IVIG), Revolade, and/or the antibodies disclosed in EP-B1 1 876 240 to said individual. The genotype can be determined as described herein.

Romiplostim (AMG 531) is an Fc fusion protein comprised of human immunoglobulin IgG1 Fc domains, with each single-chain subunit covalently linked at the C-terminus to a peptide chain containing 2 thrombopoietin (TPO) receptor-binding domains. Romiplostim may be produced by recombinant DNA technology in Escherichia coli and purified as a dimer consisting of 2 Fc-peptide-peptide subunits. It can be administered in a dose, for example, between 1.0-20.0 µg/kg dose.

### Kit

The invention also provides for prognostic or predictive assays for determining whether an individual would exhibit a positive or negative therapeutic response to the FcγRIIB therapy. The assay can be used for prognostic or predictive purpose to thereby prophylactically treat an individual prior to the treatment. The kit comprises primers to detect the Fc gamma receptor IIB 332 genotype of the individual.

The present invention further relates to a pharmaceutical package comprising an Fc gamma receptor IIB (FcγRIIB) therapy, and:
(a) instructions and/or an imprint indicating that said Fc gamma receptor IIB (FcγRIIB) therapy is to be used for the treatment of an individual (i) suffering from ITP and (ii) being characterized by the homozygous FcγRIIB 232 I/I genotype; and/or
(b) instructions and/or an imprint indicating that the individual to be treated shall be stratified.

The kits described herein can also include a program in computer readable form to analyze results of methods performed using the kits to practice the methods provided herein. The kits of the present invention may also comprise one or more of the components in any number of separate containers, packets, tubes, vials, microtiter plates and the like, or the components may be combined in various combinations in such containers. The kits of the present invention may also comprise instructions for performing one or more methods described herein and/or a description of one or more compositions or reagents described herein. Instructions and/or descriptions may be in printed form and may be included in a kit insert. A kit also may include a written description of an Internet location that provides such instructions or descriptions.

The embodiments described hereinabove, for example, with the methods of the present invention are equally applicable to the kits and uses, mutatis mutandis.

The present invention also relates to the following items:
1. A method for predicting therapeutic response to Fc gamma receptor IIB (FcγRIIB) therapy in an individual suffering from Idiopathic thrombocytopenic purpura (ITP), comprising detecting the presence of the allelic pattern for the Fc gamma receptor IIB (FcγRIIB) in a sample of said individual, wherein the presence of the homozygous FcγRIIB 232 T/T genotype is indicative of an individual that will exhibit a negative therapeutic response to said Fc gamma receptor IIB (FcγRIIB) therapy.
2. A method for predicting therapeutic response to Fc gamma receptor IIB (FcγRIIB) therapy in an individual suffering from Idiopathic thrombocytopenic purpura (ITP), comprising detecting the presence of the allelic pattern for the Fc gamma receptor IIB (FcγRIIB) in a sample of said individual, wherein the presence of the homozygous FcγRIIB 232 I/I genotype is indicative of an individual that will exhibit a positive therapeutic response to said Fc gamma receptor IIB (FcγRIIB) therapy.
3. A method of treating an individual suffering from ITP with a Fc gamma receptor IIB (FcγRIIB) therapy, wherein said individual is characterized by the homozygous FcγRIIB 232 I/I genotype, and wherein said method comprises administering an therapeutically effective amount of said Fc gamma receptor IIB (FcγRIIB) therapy to said individual.
4. Fc gamma receptor IIB (FcγRIIB) therapy for use in a method of treating an individual suffering from ITP, wherein said individual is characterized by the homozygous FcγRIIB 232 I/I genotype, and wherein said method comprises administering an therapeutically effective amount of said Fc gamma receptor IIB (FcγRIIB) therapy to said individual.
5. A method of treating an individual suffering from ITP with a Fc gamma receptor IIB (FcγRIIB) therapy, comprising detecting the presence of the allelic pattern for the Fc gamma receptor IIB (FcγRIIB) in a sample of said individual, wherein the presence of the homozygous FcγRIIB 232 I/I genotype is indicative of an individual that will exhibit a positive therapeutic response to said Fc gamma receptor IIB (FcγRIIB) therapy.
6. Fc gamma receptor IIB (FcγRIIB) therapy, for use in a method of treating an individual suffering from ITP with a Fc gamma receptor IIB (FcγRIIB) therapy, said method comprising detecting the presence of the allelic pattern for the Fc gamma receptor IIB (FcγRIIB) in a sample of said individual, wherein the presence of the homozygous FcγRIIB 232 I/I genotype is indicative of an individual that will exhibit a positive therapeutic response to said Fc gamma receptor IIB (FcγRIIB) therapy.
7. A method for stratifying individual suffering from ITP with regard to their susceptibility to treatment with a Fc gamma receptor IIB (FcγRIIB) therapy, comprising determining the allelic pattern for the Fc gamma receptor IIB (FcγRIIB) in a sample of said individual, wherein the presence of the homozygous FcγRIIB 232 T/T genotype is indicative of an individual that will exhibit a negative therapeutic response to said Fc gamma receptor IIB (FcγRIIB) therapy.
8. A method for stratifying individual suffering from ITP with regard to their susceptibility to treatment with a Fc gamma receptor IIB (FcγRIIB) therapy, comprising determining the allelic pattern for the Fc gamma receptor IIB (FcγRIIB) in a sample of said individual, wherein the presence of the homozygous FcγRIIB 232 I/I genotype is indicative of an individual that will exhibit a positive therapeutic response to said Fc gamma receptor IIB (FcγRIIB) therapy.
9. The method of item 1, 2, 3, 5, 7 or 8, wherein the Fc gamma receptor IIB (FcγRIIB) therapy is treatment with a soluble FcγRIIB receptor, romiplastin, eltrombopag, or IVIG.
10. The method of item 9, wherein the soluble FcγRIIB receptor has the amino acid sequence shown in SEQ ID No: 1 or 3.
11. The Fc gamma receptor IIB (FcγRIIB) therapy for the use of item 4 or 6, wherein the Fc gamma receptor IIB (FcγRIIB) therapy is treatment with a soluble FcγRIIB receptor, romiplastin, eltrombopag, or IVIG.
12. The Fc gamma receptor IIB (FcγRIIB) therapy for the use of item 11, wherein the soluble FcγRIIB receptor has the amino acid sequence shown in SEQ ID No: 1 or 3.

The disclosure of the present invention may best be understood in conjunction with the accompanying drawings, incorporated herein by references. Furthermore, a better understanding of the present invention and of its many advantages will be had from the following examples, given by way of illustration and are not intended as limiting.

The present invention also relates to the following clauses:
1. A method for predicting therapeutic response to Fc gamma receptor IIB (FcγRIIB) therapy in an individual suffering from Idiopathic thrombocytopenic purpura (ITP), comprising detecting the presence of the allelic pattern for the Fc gamma receptor IIB (FcγRIIB) in a sample of said individual, wherein the presence of the homozygous FcγRIIB 232 T/T genotype is indicative of an individual that will exhibit a negative therapeutic response to said Fc gamma receptor IIB (FcγRIIB) therapy.
2. A method for stratifying individual suffering from ITP with regard to their susceptibility to treatment with a Fc gamma receptor IIB (FcγRIIB) therapy, comprising determining the allelic pattern for the Fc gamma receptor IIB (FcγRIIB) in a sample of said individual, wherein the presence of the homozygous FcγRIIB 232 T/T genotype is indicative of an individual that will exhibit a negative therapeutic response to said Fc gamma receptor IIB (FcγRIIB) therapy.
3. A method for stratifying individual suffering from ITP with regard to their susceptibility to treatment with a Fc gamma receptor IIB (FcγRIIB) therapy, comprising determining the allelic pattern for the Fc gamma receptor IIB (FcγRIIB) in a sample of said individual, wherein the presence of the homozygous FcγRIIB 232 I/I genotype is indicative of an individual that will exhibit a positive therapeutic response to said Fc gamma receptor IIB (FcγRIIB) therapy.
4. Fc gamma receptor IIB (FcγRIIB) therapy for use in a method of treating an individual suffering from ITP, wherein said individual is characterized by the homozygous FcγRIIB 232 I/I genotype, and wherein said method comprises administering an therapeutically effective amount of said Fc gamma receptor IIB (FcγRIIB) therapy to said individual.
5. Fc gamma receptor IIB (FcγRIIB) therapy, for use in a method of treating an individual suffering from ITP with a Fc gamma receptor IIB (FcγRIIB) therapy, said method comprising detecting the presence of the allelic pattern for the Fc gamma receptor IIB (FcγRIIB) in a sample of said individual, wherein the presence of the homozygous FcγRIIB 232 I/I genotype is indicative of an individual that will exhibit a positive therapeutic response to said Fc gamma receptor IIB (FcγRIIB) therapy.
6. The method of clause 1, 2, or 3, wherein the Fc gamma receptor IIB (FcγRIIB) therapy is treatment with a soluble FcγRIIB receptor, romiplastin, eltrombopag, or IVIG.
7. The method of clause 6, wherein the soluble FcγRIIB receptor has the amino acid sequence shown in SEQ ID No: 1 or 3.
8. The Fc gamma receptor IIB (FcγRIIB) therapy for the use of clause 4 or 5, wherein the Fc gamma receptor IIB (FcγRIIB) therapy is treatment with a soluble FcγRIIB receptor, romiplastin, eltrombopag, or IVIG.
9. The Fc gamma receptor IIB (FcγRIIB) therapy for the use of clause8, wherein the soluble FcγRIIB receptor has the amino acid sequence shown in SEQ ID No: 1 or 3.

## Claims

1. A soluble Fc gamma receptor IIB (FcγRIIB) for use in a method of treating an individual suffering from ITP, wherein said individual is **characterized by** the homozygous FcγRIIB 232 I/I genotype, wherein I is isoleucine, and wherein said method comprises administering a therapeutically effective amount of said soluble Fc gamma receptor IIB (FcγRIIB) to said individual.

2. A soluble Fc gamma receptor IIB (FcγRIIB) for use in a method of treating an individual suffering from ITP, said method comprising detecting the presence of the allelic pattern for Fc gamma receptor IIB (FcγRIIB) in a sample of said individual, wherein the presence of the homozygous FcγRIIB 232 I/I genotype, wherein I is isoleucine, is indicative of an individual that will exhibit a positive therapeutic response to said soluble Fc gamma receptor IIB (FcγRIIB).

3. The soluble Fc gamma receptor IIB (FcγRIIB) for the use according to claim 1 or 2, wherein the soluble Fc gamma receptor IIB (FcγRIIB) consists of the amino acid sequence shown in SEQ ID No: 1 or 3.


SEQUENCE LISTING

<110>  SuppreMol GmbH

<120>  Stratification and Treatment of Patients of Idiopathic
       Thrombocytopenic Purpura

<130>  SUP14626EP-A

<150>  EP 12 008 209.4
<151>  2012-12-07

<160>  4

<170>  PatentIn version 3.5

<210>  1
<211>  181
<212>  PRT
<213>  artificial

<220>
<223>  Fc gamma receptor II B variant

<400>  1

Met Gly Thr Pro Ala Ala Pro Pro Lys Ala Val Leu Lys Leu Glu Pro
1               5                   10                  15

Gln Trp Ile Asn Val Leu Gln Glu Asp Ser Val Thr Leu Thr Cys Arg
            20                  25                  30

Gly Thr His Ser Pro Glu Ser Asp Ser Ile Gln Trp Phe His Asn Gly
        35                  40                  45

Asn Leu Ile Pro Thr His Thr Gln Pro Ser Tyr Arg Phe Lys Ala Asn
    50                  55                  60

Asn Asn Asp Ser Gly Glu Tyr Thr Cys Gln Thr Gly Gln Thr Ser Leu
65                  70                  75                  80

Ser Asp Pro Val His Leu Thr Val Leu Ser Glu Trp Leu Val Leu Gln
                85                  90                  95

Thr Pro His Leu Glu Phe Gln Glu Gly Glu Thr Ile Val Leu Arg Cys
            100                 105                 110

His Ser Trp Lys Asp Lys Pro Leu Val Lys Val Thr Phe Phe Gln Asn
        115                 120                 125

Gly Lys Ser Lys Lys Phe Ser Arg Ser Asp Pro Asn Phe Ser Ile Pro
    130                 135                 140

Gln Ala Asn His Ser His Ser Gly Asp Tyr His Cys Thr Gly Asn Ile

145                 150                 155                 160

Gly Tyr Thr Leu Tyr Ser Ser Lys Pro Val Thr Ile Thr Val Gln Ala
                165                 170                 175

Pro Ser Ser Ser Pro
            180

<210>  2
<211>  543
<212>  DNA
<213>  artificial

<220>
<223>  Fc gamma receptor II B variant

<400>  2
atggggacac ctgcagctcc cccaaaggct gtgctgaaac tcgagcccca gtggatcaac      60

gtgctccagg aggactctgt gactctgaca tgccggggga ctcacagccc tgagagcgac     120

tccattcagt ggttccacaa tgggaatctc attcccaccc acacgcagcc cagctacagg     180

ttcaaggcca acaacaatga cagcggggag tacacgtgcc agactggcca gaccagcctc     240

agcgaccctg tgcatctgac tgtgctttct gagtggctgg tgctccagac ccctcacctg     300

gagttccagg agggagaaac catcgtgctg aggtgccaca gctggaagga caagcctctg     360

gtcaaggtca cattcttcca gaatggaaaa tccaagaaat tttcccgttc ggatcccaac     420

ttctccatcc cacaagcaaa ccacagtcac agtggtgatt accactgcac aggaaacata     480

ggctacacgc tgtactcatc caagcctgtg accatcactg tccaagctcc cagctcttca     540

ccg                                                                   543

<210>  3
<211>  177
<212>  PRT
<213>  artificial

<220>
<223>  Fc gamma receptor II B variant

<400>  3

Met Ala Pro Pro Lys Ala Val Leu Lys Leu Glu Pro Gln Trp Ile Asn
1               5                   10                  15

Val Leu Gln Glu Asp Ser Val Thr Leu Thr Cys Arg Gly Thr His Ser
            20                  25                  30

Pro Glu Ser Asp Ser Ile Gln Trp Phe His Asn Gly Asn Leu Ile Pro
        35                  40                  45

Thr His Thr Gln Pro Ser Tyr Arg Phe Lys Ala Asn Asn Asn Asp Ser
    50                  55                  60

Gly Glu Tyr Thr Cys Gln Thr Gly Gln Thr Ser Leu Ser Asp Pro Val
65                  70                  75                  80

His Leu Thr Val Leu Ser Glu Trp Leu Val Leu Gln Thr Pro His Leu
                85                  90                  95

Glu Phe Gln Glu Gly Glu Thr Ile Val Leu Arg Cys His Ser Trp Lys
            100                 105                 110

Asp Lys Pro Leu Val Lys Val Thr Phe Phe Gln Asn Gly Lys Ser Lys
        115                 120                 125

Lys Phe Ser Arg Ser Asp Pro Asn Phe Ser Ile Pro Gln Ala Asn His
    130                 135                 140

Ser His Ser Gly Asp Tyr His Cys Thr Gly Asn Ile Gly Tyr Thr Leu
145                 150                 155                 160

Tyr Ser Ser Lys Pro Val Thr Ile Thr Val Gln Ala Pro Ser Ser Ser
                165                 170                 175

Pro

<210>  4
<211>  534
<212>  DNA
<213>  artificial

<220>
<223>  Fc gamma receptor II B variant

<400>  4
atggcaccgc cgaaagcagt tctgaaactg gaaccgcagt ggattaacgt tctgcaggaa      60

gatagcgtta ccctgacctg tcgtggcacc catagcccgg aaagcgatag cattcagtgg     120

tttcacaacg gcaatctgat tccgacccat acccagccga gctatcgttt taaagcgaac     180

aacaacgata gcggcgaata tacctgtcag accggtcaga ccagcctgag cgatccggtt     240

catctgaccg ttctgagcga atggctggtt ctgcagaccc cgcatctgga atttcaggaa     300

ggcgaaacca ttgttctgcg ttgccacagc tggaaagata aaccgctggt taaagttacc     360

ttcttccaga acggcaaaag caaaaaattc agccgtagcg atccgaattt tagcattccg     420

caggcgaatc atagccatag cggcgattat cattgtaccg gcaacattgg ctataccctg     480

tatagcagca aaccggtgac cattaccgtt caggcgccga gcagcagccc gtaa           534